# EUROPEAN PATENT SPECIFICATION

(11) **EP 2 575 840 B1**
(45) Date of publication and mention of the grant of the patent: **21.03.2018**
(21) Application number: 11731068.0
(22) Date of filing: 25.05.2011
(51) Int. Cl.: A61K 36/47, A61K 36/61, A61P 17/02

(54) **KIT OF PARTS FOR TREATING AND/OR PREVENTING CUTANEOUS ULCERS**
KIT OF PARTS ZUR BEHANDLUNG/VORBEUGUNG VON HAUTGESCHWÜREN
TROUSSE DE PIÈCES POUR LE TRAITEMENT ET/OU LA PRÉVENTION D'ULCÈRES CUTANÉS

(30) Priority: 27.05.2010 IT MI20100953
(43) Date of publication of application: 10.04.2013
(73) Proprietor: Logi, Roberto, 50124 Firenze (IT)
(72) Inventor: Logi, Roberto, 50124 Firenze (IT)
(74) Representative: Brazzini, Silvia
(86) International application number: PCT/IB2011/001137
(87) International publication number: WO 2011/148257

(56) References cited:
- US-A1- 2004 151 710
- US-A1- 2007 104 728
- CARPENTER M R ET AL: "Antibacterial effects of Sangre de Grado on Gram-positive and Gram-negative bacteria", ABSTRACTS OF THE GENERAL MEETING OF THE AMERICAN SOCIETY FOR MICROBIOLOGY, THE SOCIETY, WASHINGTON, DC, US, vol. 101, 24 May 2001 (2001-05-24), page 38, XP009141691, ISSN: 1060-2011

## Description

The present invention refers to a kit of parts for use in treating and/or preventing cutaneous ulcers, preferably chronic ulcers, such as for example decubitus ulcers, more preferably trophic ulcers of the lower limbs, and for treating and/or preventing cutaneous ulcers caused by the *Herpes simplex* virus, preferably lip ulcers, or cutaneous ulcers that occur in form of *Herpes zoster,* commonly referred to as shingles.

Cutaneous ulcers are solutions of continuity affecting the skin and, at times, subcutaneous tissue, with slow reparation, caused by trauma, infections or, more often, vascular or neuropathic trophic disorders. The shape, dimensions, related symptoms usually allow easy diagnosis (Di Stanislao C., Ciarfella R. "Terapia combinata in corso di ulcere trofiche vasculopatiche dell'arto inferiore refrattarie ad altri trattamenti" Yi Dao Za Zhi, 2002 (vol. 18*)*, only online).

There are various cure strategies for preventing and treating cutaneous ulcers, in particular the chronic ones, i.e. lesions that take more than 6 weeks to heal. Most of these are leg ulcers, diabetic foot, decubitus sores and the surgical wounds which open suffering infections. The management of chronic lesions (cutaneous ulcers) usually requires local treatment, for example by reducing the pressure and treating the surroundings of the lesion: bandages, pressure release-beds, use of mattresses and cushions are universally known solutions (Cullum N. et al, "Systematic reviews of wound care management: (5) beds; (6) compression; (7) laser therapy, therapeutic ultrasound, electrotherapy and electromagnetic therapy", Health Technology Assessment (2001); vol 5: No. 9)*.*

Cullum *et al.* indicates how soothing techniques through releasing pressure and compression are effective in the cutaneous ulcer therapy, while stressing how there are no particular improvements as regards with the chronic lesions for techniques such as laser therapy, therapeutic ultrasound, electrotherapy and electromagnetic therapy.

For the prevention and treatment of topic cutaneous ulcers, in particular venous ulcers, there can be used various methods of compression through bandages and occlusive medications, semi-occlusive medications, absorbents, medications based on carboxymethylcellulose, alginates, polyurethane, collagen, fibrin glue, chitosan, in form of pastes, granules, foams and gels, depending on the development stage of the lesion itself (necrotic, fibrinous exudated, infected, deterged, granulating, reepithelizing). The local application of growth factors has been recently proposed (ECM course "le ulcere trofiche degli arti inferiori: percorsi terapeutici fra tradizione e innovazione" - *Asl*/*Rome* - *"Sandro Pertini" Hospital 18th April 2009*)*.*

In case of an infection, cultures of the exudate of the ulcer should be prepared and a treatment using systemic antibiotics should be carried out. Antibiotics for topic use are generally not used, due to the fact that they can facilitate the occurrence of contact dermatitis.

Venous ulcer surgery should be considered complementary to the conservative treatment and it can be aimed at correcting the basic haemodynamic alteration or covering the ulcer by means of cutaneous grafts with the aim of reducing the healing times.

It is commonly believed that in patients with varicose ulcer, surgery of the surface venous system offers optimal results, reducing the healing times and relapse over time, especially in absence of alterations of the deep venous system. Surgical results in case of post-thrombotic ulcers have yielded poor results.

Furthermore, though revealing to have drastically reduced morbidity, the endoscopic technique compared with the open technique for an approximately four-year follow-up did not reveal statistically significant differences in terms of healing the ulcers.

Operations such as valvuloplasty, transplants of venous valve and venous transposition operations can be considered as the last solution.

Regarding cutaneous grafts, also literature does not provide sufficient evidence of the effects thereof on the stable healing of venous ulcers. They can be implemented through various methods, such as "meshed split skin grafting"; "pinch grafting"; homotransplant of in vitro cultured human keratinocytes; free flap graft with valvular venous segments, after ulcerectomy and binding of insufficient perforans; "shave therapy", i.e. ulcerectomy, removal of the lipodermatosclerotic tissue and "meshed" grafting (ECM course- *ASL*/*Rome - "Sandro Pertini" hospital, see supra).*

The local ulcer therapy should be accompanied by an elastic compression therapy and by the systemic medical therapy where required (targeted antibiotic therapy, vasoactive hemorheologics, antithrombotica, antiphlogistics, etc).

In addition, in order to further improve the result and to obtain a greater compliance by the patient, it is necessary to provide personalized antalgic therapy, in that the presence of a cutaneous ulcer may cause nociceptive pain and neuropathic pain. The term nociceptive pain is used to indicate a physiological response to chronic or acute tissue damaging inflammation, generally limited over time. When the wounds heal slowly, extended inflammatory response may lead to greater sensitivity, both at wound level (primary hyperalgesia) and at the surrounding skin (secondary hyperalgesia). The term neuropathic pain is used to indicate an inappropriate response caused by a primary lesion or dysfunction of the nervous system. Nerve damage is the most common cause of primary lesion which can be caused by trauma, infection, metabolic dysfunction or cancer.

In view of what has been described above, it can be concluded that, regardless of the wide variety of medications proposed, there still lacks an ideal therapy and strict protocols valid for the cure of all cutaneous ulcers, in particular venous ulcers, cannot be drafted.

Thus, there is still the need for a cutaneous ulcer therapy capable of allowing the cicatrisation of said ulcers within quick times, soothing pain, stimulating growth of the granulation tissue, while creating a barrier against bacteria and mycets.

Lip herpes (*herpes labialis*) is an infective disease which generally affects the lips, where ulcers occur. This particular type of herpes is caused by a viral strain called *Herpes simplex* (usually of type I). At times, the *Herpes simplex* infection can cause the occurrence of lesions even on the cheeks, nose and palate.

The symptoms of the disease are characteristic and share the occurrence of annoying and painful small blisters (ulcers), while before the occurrence of the small blisters, the affected area is preceded by warmth, tension and tingling sensation, clearly perceptible by those already affected by the disease.

Lip herpes is usually treated with topical antivirals, generally acyclovir-based ointments, obtaining complete healing within about 5-7 days from the occurrence of the small blisters. However, such active ingredient is effective in preventing the occurrence of the small blisters only if administered at the initial stages of the reactivation of the virus, i.e. upon feeling the tingling sensation typical of herpes infection. Topical or systemic antivirals used in the most serious cases, are symptomatic treatments, in that the virus remains in the organism, and thus relapse is frequent, in the presence of trigger factors such as, for example, intense heat/cold, exposure to sunlight, physical fatigue.

Thus, there clearly arises the need for a medicament capable of optimally curing the herpes infection, even when it has been diagnosed, due to the presence of blisters. Such medicament should be capable of accelerating the healing times with respect to the conventional antivirals and preventing relapse.

*Herpes zoster,* commonly referred to as shingles, is an extremely painful disease, affecting the skin and nervous terminations, caused by the chickenpox virus (*varicella-zoster virus*). The formation of blisters and crusts on the skin is accompanied by acute pain along the affected nerve, which may last over a long period of time. It usually occurs on the chest or on the stomach, more rarely on the face.

Cure comprises ointments and lotions applied locally, cutaneous protection with sterile bandages, antihistaminics. Oral antibiotics are useful only if there are concurrent infections, and painkillers, ranging from aspirin to the more potent ones sooth the pain of the patient.

Local infiltration of anaesthetics or creams as well as anaesthetic patches can be used in case of extremely strong pain. There are antiviral drugs (such as aciclovir and valaciclovir and famciclovir) which can, when taken early, accelerate healing and help preventing post-herpetic neuralgia.

It has been surprisingly found that the combined use of a *Croton lechleri* resin extract and *Melaleuca alternifolia* oil, allows an extremely quick cicatrisation of cutaneous ulcers, in particular chronic ulcers, such as for example decubitus ulcers, even more in particular trophic ulcers of the lower limbs, with the ensuing complete healing of said lesions.

Furthermore, it has been surprisingly found that the combined use as previously described allows resolving, within quick times, cutaneous ulcers caused by the *Herpes simplex* virus, in particular lip ulcers, also preventing relapse thereof, or the cutaneous ulcers that occur in form of *Herpes zoster.*

*Croton Lechleri* (*Euphorbiaceae*), is a tree that grows in the low mountain areas of the Andine regions of Peru, as well as in Colombia, Ecuador and Bolivia, and it is known for its therapeutic properties. The cortex, upon cutting, releases a reddish or yellowish latex called "dragon's blood" used by the indigenous tribes for medical purposes, to stop bleeding, help healing wounds and for intestinal diseases therapy.

This red resin was also used for varnishing wood, as incense and as dye (Williams J.E., Altern Med Rev (2001), vol6 (6),p.567-579).

*Melaleuca* is a plant genus of the Myrtaceae family, of which there are over 200 known species, most of which are endemic in Australia, Malaysia and New Caledonia.

*Melaleuca alternifolia* is a known species of Melaleuca, important for its essential oil (*Teatree oil*). This oil has antifungal and antibiotic properties, used at topical level (Carson et al., Antimicrob. Agents Chemother.(2002) vol. 46,(6) p. 1914-1920).

It has been surprisingly found that the combined use of a *Croton lechleri* resin extract and *Melaleuca alternifolia* oil has an improved synergistic effect with respect to the use of the aforementioned single components, in the cicatrisation of cutaneous ulcers, in particular chronic ulcers, such as decubitus ulcers, more in particular trophic ulcers of the lower limbs. With the use of said kit, there is actually observed an advantageous increase of the speed of cicatrisation of cutaneous ulcers, in particular chronic ulcers, such as decubitus ulcers, and even more in particular trophic ulcers of the lower limbs, with the ensuing complete healing of said lesions.

Furthermore, it has been surprisingly found that the previously described combined use has an improved synergistic effect with respect to the use of known antivirals generally used (such as aciclovir), for complete healing - within short periods of time, i.e. 1-4 days, preferably 1-3 days and even more preferably 1-2 days - of cutaneous ulcers, in particular lip ulcers, caused by the *Herpes simplex* virus, also preventing relapse thereof.

Additionally, it has been surprisingly found that the previously described combined use has an improved synergistic effect with respect to the use of known antivirals generally used (such as aciclovir, valaciclovir and famciclovir), for the complete healing - within a short period of time - of cutaneous ulcers that occur in form of *Herpes zoster.*

Optionally, one or more elasticisers can be used alongside the *Croton lechleri* resin extract and *Melaleuca alternifolia* oil. Said elasticisers are preferably selected from among an ointment based on *Calendula officinalis,* and/or an ointment or cream based on *Hamamelis virginiana,* and/or *Hypericum perforatum* oil.

*Calendula* is a plant of the *Asteraceae* genus, native of Europe, North Africa and Middle Eastern Asia, comprising more than 20 annual or perennial herbaceous species, the most renowned of which is *Calendula officinalis.* The use of its flowers for medication purposes, has antispasmodic and cicatrizing effects: actually the decoction is recommended for gastric ulcer. Furthermore, it has sudoriferous effects and prevention/soothing effects on menstrual pains. Regarding the menstrual cycle it also has a reduction effect on the flow and a regularization effect. It is at times used in antihistaminic products to treat allergies caused by dust and mites. In dermocosmetics there is produced a cream using the powder obtained from dry flowers, having effects against acne and skin spots ("Aromatiche, belle buone e di molte virtù"-Camera di Commercio Industria e Artigianato di Savona - (2003) p. 59 - 62).

*Hamamelis virginiana L.* is a plant of the *Hamamelidaceae* genus, native from the East of the North American continent (Quebec, Virginia, Florida), which can be cultivated, for ornamental purposes, even in Europe. The leaves and the cortex of the caulis thereof are picked. The leaves of hamamelis are used both for the preparation of conventional phytotherapy, indicated in the haemorrhoidal symptoms, and for treating occurrence of venous insufficiency (Medicalia, (2003) vol. 68, p. 1 - Ed. Omeosalus).

The *Hypericum* genus comprises numerous garden species, such as *H. androsaemum, H. calcynum, H. moserianum* and others, all having yellow flowers; among many others *Hypericum perforatum* is probably the most known one due to its magical and medicinal effects. The flowering tops of Hypericum are used. Over the years, the plant has been attributed antiseptic, analgesic, anti-catarrhal, antidepressant, antiphlogistic, antispasmodic, astringent, anti-diarrhoea, balsamic, cholagogue, stimulation of the bile secretion, decongestive, digestive, diuretic, tonic, sedative, vermifuge and vulnerary properties for the cicatrisation of wounds ("Aromatiche, belle buone e di molte virtu" - Camera di Commercio Industria e Artigianato di Savona - (2003) p. 89 - 92).

Thus, an object of the present invention relates to a kit of parts for use in treating and/or preventing cutaneous ulcers, in particular chronic ulcers such as decubitus ulcers, in particular trophic ulcers of the lower limbs, comprising a *Croton lechleri* resin extract and *Melaleuca alternifolia* oil.

Object of the present invention is also a kit of parts for use in treating and/or preventing cutaneous ulcers, in particular lip ulcers, caused by the *Herpes simplex* virus, or cutaneous ulcers that occur in form of *Herpes zoster,* comprising a *Croton lechleri* resin extract *and Melaleuca alternifolia* oil.

Optionally, alongside *Croton lechleri* resin extract and *Melaleuca alternifolia* oil, in the previously described kit of parts for use in treating and/or preventing cutaneous ulcers, there can also be provided elasticisers, such as an ointment or cream based on *Calendula officinalis* and/or an ointment or cream based on *Hamamelis virginiana,* and/or *Hypericum perforatum* oil.

Object of the present invention is also a kit of parts comprising a *Croton lechleri* resin extract *and Melaleuca alternifolia* oil, for use as a medicament in a patient having cutaneous ulcers, such as decubitus ulcers, in particular trophic ulcers of the lower limbs.

A further object of the present invention is a kit of parts comprising a *Croton lechleri* resin extract and *Melaleuca alternifolia* oil for use as a medicament in a patient having cutaneous ulcers, in particular lip ulcers, caused by the *Herpes simplex* virus, or cutaneous ulcers that occur in form of *Herpes zoster.*

Optionally, alongside *Croton lechleri* resin extract and *Melaleuca alternifolia* oil, comprised in the previously described kit of parts for use as a medicament, there can be further provided elasticisers, such as an ointment or cream based on *Calendula officinalis,* and/or an ointment or cream based on *Hamamelis virginiana,* and/or *Hypericum perforatum* oil.

Preferably, the *Croton lechleri* resin extract used in the kit of parts subject of the present invention is a liquid solution of resinous latex, 100% of plant origin, of the Croton Lechleri plant at least 5 years old, and even more preferably it is a liquid solution of pure *Croton lechleri* resin extracted in 10% of alcohol or it is a liquid solution of pure *Croton lechleri* resin in 1:10 dilution in hydro-glycero-alcoholic solution.

Preferably, the *Melaleuca alternifolia* oil used in the kit of parts subject of the present invention is a 100% essential oil.

The elasticisers of the present invention are used according to the needs up to complete healing.

Preferably, the ointment or cream based on *Calendula officinalis* used in the kit of parts subject of the present invention contains a hydroalcoholic extract comprised between 5 and 15% of *Calendula officinalis,* preferably it contains a hydroalcoholic extract at 10% of *Calendula officinalis.*

Preferably, the ointment or cream based on *Hamamelis virginiana* contains *Hamamelis virginiana* in a dilution comprised between 1DH and 8DH, preferably between 2DH and 6DH and even more preferably at 4DH.

Preferably, the *Hypericum perforatum* oil is a 100% essential oil

Preferably, the *Croton lechleri* resin extract and the *Melaleuca alternifolia* oil used in the kit of parts subject of the present invention, are used at a ratio comprised between 1:1.5 and 1:3, preferably at a ratio comprised between 1:1.6 and 1:2.2, and even more preferably the ratio thereof is 1:2.

Preferably, the amount in weight of *Croton lechleri* resin administered daily on the ulcer is comprised between 60 and 600 mg, and/or the amount expressed in volume for the daily application on the ulcer, is comprised between 0.20 and 2 ml, preferably between 0.5 and 1.5 ml daily.

Not only does the increase of the amount of *Croton lechleri* exceeding the previously described ratios with the *Melaleuca alternifolia* oil, not improve the cicatrizing effect of the kit it also reduces its effect.

The term "kit of parts" as used herein indicates a combined preparation containing, as active ingredients, *Croton lechleri* resin extract and *Melaleuca alternifolia* oil for use simultaneously, separately or sequentially, for treating and/or preventing cutaneous ulcers, in particular chronic ulcers, such as decubitus ulcers, more in particular trophic ulcers of the lower limbs, or for use simultaneously, separately or sequentially, for treating and/or preventing cutaneous ulcers, in particular lip ulcers, caused by the *Herpes simplex* virus, or cutaneous ulcers that occur in form of *Herpes zoster.*

Optionally, said "kit of parts" may comprise one or more elasticisers, such as for example an ointment or cream based on *Calendula officinalis,* and/or an ointment or cream based on *Hamamelis virginiana,* and/or *Hypericum perforatum* oil.

The two individual ingredients of the kit of parts, i.e. *Croton lechleri* resin extract and the *Melaleuca alternifolia* oil (also referred to as "the two active ingredients"), form a functional unit, i.e. an actual functional combination through a direct application for the purpose. Due to the use thereof in the kit of parts of the present invention, the two active ingredients show a synergistic effect.

Advantageously, the administration by application of the kit of parts of the present invention on the cutaneous ulcers, in particular on chronic ulcers, such as decubitus ulcers, and even more in particular on the trophic ulcers of the lower limbs occurs several times a day, i.e. 3-5 times, preferably two times a day, even more preferably in the morning and in the evening.

Advantageously, the administration by application of the kit of parts of the present invention on the cutaneous ulcers, in particular lip ulcers, caused by the *Herpes simplex* virus, and on ulcers that occur in form of *Herpes zoster,* occurs preferably several times a day, i.e. 3-5 times, preferably two times a day, preferably in the morning and in the evening.

Advantageously, the administration by application of the kit of parts of the present invention on the cutaneous ulcers, in particular on chronic ulcers, such as decubitus ulcers and even more in particular on trophic ulcers of the lower limbs continues up to complete healing of the ulcers, which occurs within a time interval lasting from 1 week to 4 months from the beginning of the treatment, depending on the seriousness of the lesion. Preferably, complete healing occurs within a time interval lasting from 2 weeks to 3 months and even more preferably within a time interval lasting from 3 weeks to 2 months from the beginning of the treatment.

Advantageously, the administration by application of the kit of parts of the present invention on the cutaneous ulcers, in particular lip ulcers, caused by the *Herpes simplex* virus continues up to complete healing of the ulcers, which occurs within 1-4 days from the beginning of the treatment. Preferably, complete healing occurs within 1-3 days and even more preferably within 1-2 days, without further relapse.

In another advantageous combined preparation according to the invention, the kit of parts of the present invention is applied on the cutaneous ulcers, such as decubitus ulcers, in particular on the trophic ulcers of the lower limbs or on the cutaneous ulcers, in particular lip ulcers, caused by the *Herpes simplex* virus, or in the ulcers that occur in form of *Herpes zoster,* simultaneously.

In another advantageous combined preparation according to the invention, the kit of parts is applied on the cutaneous ulcers, such as decubitus ulcers, in particular on trophic ulcers of the lower limbs or on the cutaneous ulcers, in particular lip ulcers, caused by the *Herpes simplex* virus, or in ulcers that occur in form of *Herpes zoster,* sequentially, *Croton lechleri* being preferably applied before *Melaleuca alternifolia* oil.

In another advantageous combined preparation according to the invention, the kit of parts is applied on the cutaneous ulcers, such as decubitus ulcers, in particular on trophic ulcers of the lower limbs or on the cutaneous ulcers, in particular lip ulcers, caused by the *Herpes simplex* virus, or on ulcers that occur in form of *Herpes zoster,* within a time interval between the application of the *Croton lechleri* resin and the application of the *Melaleuca alternifolia* oil, which can preferably be comprised between one day and one week.

In another advantageous combined preparation according to the invention, the kit of parts is applied on ulcers or cutaneous wounds of non-human mammals such as felines, canids and equines.

The active ingredients of the kit of parts of the present invention are administered both simultaneously and separately or sequentially according to the present invention, and they represent a new combination having the surprising properties described above, and not a mere aggregate of known agents.

It should be observed that, the combined preparation designated as the kit of parts does not imply that the components of the combined preparation are necessarily present blended, for example a composition, so as to be available for separate or sequential application.

### Brief description of the figures

Figure 1 - Ulcerous trophic lesion after 4 days of treatment with the kit of the present invention, in the presence of *Calendula officinalis* as optional elasticiser (date: 23^{rd} March 2010)
Figure 2 - Ulcerous trophic lesion after 29 days of treatment using the kit of the present invention in the presence of *Calendula officinalis* as optional elasticiser (date 17^{th} April 2010)
Figure 3 - Ulcerous trophic lesion after 49 days of treatment using the kit of the present invention in the presence of *Calendula officinalis* as optional elasticiser (date 7^{th} May 2010)
Figure 4 - Ulcerous trophic lesion healed after 55 days of treatment using the kit of the present invention in the presence of *Calendula officinalis* as optional elasticiser (date 13^{th} May 2010)
Figure 5 - Ulcerous trophic lesion completely healed after 62 days of treatment using the kit of the present invention in the presence of *Calendula officinalis* as optional elasticiser (date 20^{th} May 2010)
Figure 6 - Ulcerous trophic lesion after 5 days of treatment using the kit of the present invention, in the presence of *Hypericum perforatum* as optional elasticiser (date 24^{th} March 2010)
Figure 7 - Ulcerous trophic lesion after 9 days of treatment using the kit of the present invention in the presence of *Hypericum perforatum* as optional elasticiser (date 28^{th} March 2010)
Figure 8 - Ulcerous trophic lesion after 29 days of treatment using the kit of the present invention in the presence of *Hypericum perforatum* as optional elasticiser (date 17^{th} April 2010)
Figure 9 - Ulcerous trophic lesion healed after 55 days of treatment using the kit of the present invention in the presence of *Hypericum perforatum* as optional elasticiser (date 13^{th} May 2010)

### Examples

### Example 1 - clinical case of trophic ulcer treated using the kit of the present invention in the presence of Calendula officinalis as optional elasticiser

An 86 year-old overweight female subject, affected by chronic lymphatic leukaemia, had the following clinical record:
- occurrence of a trophic ulcer on the right lower limb in September 2009, treated conventionally, i.e. using enzymatic ointments, bandages, gauzes and patches;
- deterioration of the trophic ulcer requiring urgent hospitalisation on 24^{th} December 2009. Here, the patient is treated using enzymatic ointments and elastic-compression bandage, alongside anticoagulant treatment (dalteparin sodium, platelet antiaggregants (ticlopidine), anti-anginals (nitroglycerine), diuretic drugs (lansoprazole) and local painkillers (fentanyl transdermal patches) and systemic painkillers (paracetamol and codeine phosphate), also due to the general clinical record. Regardless of the diagnosis of "obliterating arteriopathy of the lower limbs with pretibial trophic ulcer", the subject was discharged on 15^{th} January 2010.

- On 1^{st} February 2010, angioplasty of the right common iliac artery is attempted, but it is ineffective.
- On 1^{st} March 2010, following further medical checkup, new angioplasty is rescheduled (recanalisation of the right iliac through percutaneous method) so as to avoid gangrene and avoid the amputation of the limb affected with trophic ulcer.
- On 12^{th} March 2010 the conditions of the subject deteriorate due to secondary diseases (strong rectorrhagia) and new hospitalization is required, where, with respect to the treatment scheme of the 15^{th} January 2010, ticlopidine is eliminated and dalteparin sodium is reduced. The subject is discharged on 19^{th} March 2010, without considerable benefits with respect to the trophic ulcer.

On this date, i.e. 19^{th} March 2010, the clinical experimentation using the kit of parts of the present invention was commenced.

After eliminating the bandages arranged on the trophic ulcer, the wound was washed using saline solution and it was sprinkled with drops of *Croton lechleri* resin extract i.e. pure *Croton lechleri* resin extracted in 10% of alcohol, leaving the trophic ulcer uncovered, without bandages. A crust had already formed after 24 hours and the re-cicatrisation process had already begun after 48 hours. The strong pains on the leg ceased and the patient stopped using further analgesics.

Drops of *Melaleuca alternifolia* essential oil at 100%, at an amount of drops about twice the amount of *Croton lechleri* extract, were applied around the ulcer thus obtaining an anti-itching lenitive effect, alongside acceleration of the re-epithelization process of the trophic ulcer.

Lastly, an ointment based on *Calendula officinalis,* comprising a hydroalcoholic extract at 10% of *Calendula officinalis,* was applied on the re-epithelized part so as to improve the elasticity of the new epithelium.

The two ingredients of the kit of the present invention, as well as the ointment based on *Calendula officinalis,* were applied twice a day, in the morning and in the evening, where each application consisted of about 0.25-0.75 ml of *Croton lechleri* extract, 0.25 ml of *Melaleuca alternifolia,* 1.5 g of ointment based on *Calendula officinalis.*

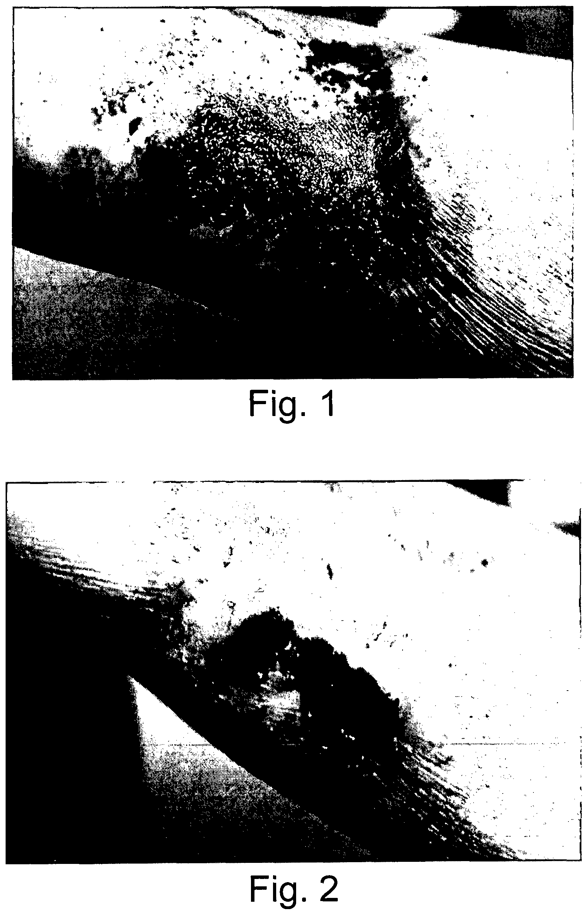

After about four days from the begenning of the treatment, i.e. on 23^{rd} March 2010, there was observed the formation of hard crust on the trophic lesion, as outlined in figure 1.

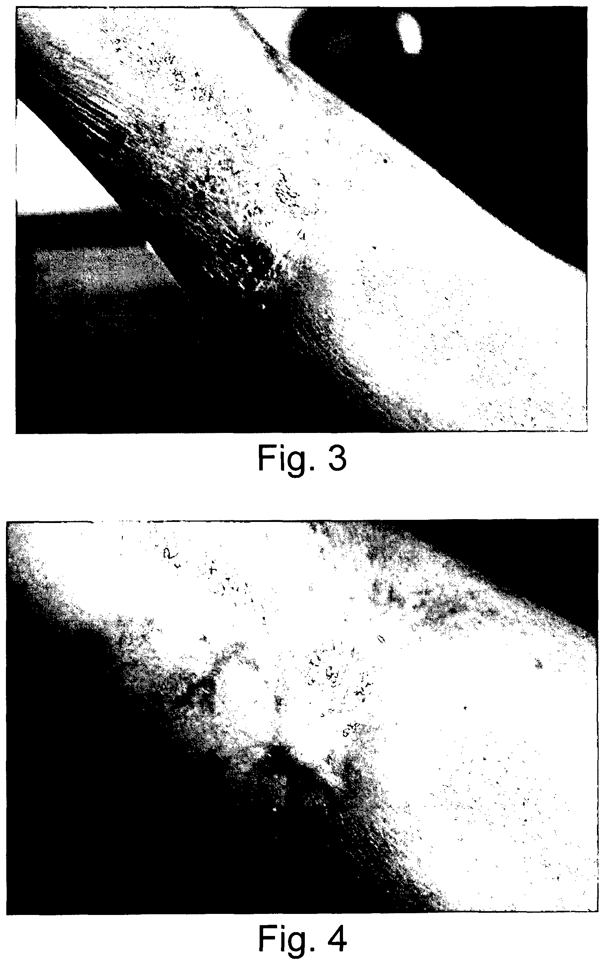

After about 29 days of treatment using the kit of the present invention, alongside the application of an ointment based on *Calendula officinalis,* comprising a hydroalcoholic extract at 10% of *Calendula officinalis* (date 17^{th} April 2010, figure 2), the extension of the trophic lesion appeared considerably reduced and after 49 days of treatment (date 7^{th} May 2010, figure 3), the trophic ulcer appeared almost entirely healed.

As shown in figure 4, after 55 days of treatment (13^{th} May 2010), the trophic lesion was almost entirely healed.

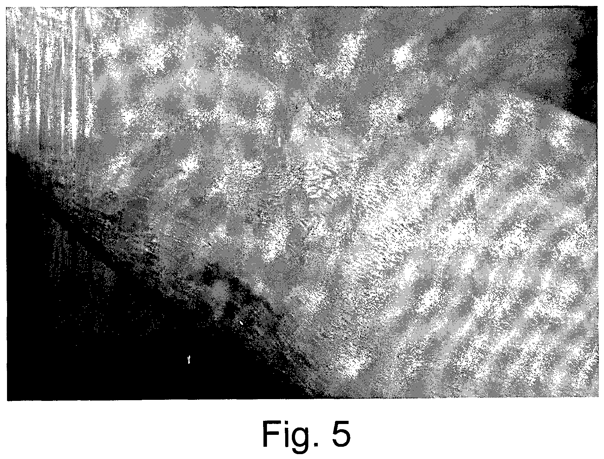

Lastly, as shown in figure 5, the ulcerous trophic lesion appears entirely healed after 62 days of treatment using the kit of the present invention, in the presence of *Calendula officinalis* as optional elasticiser (date 20^{th} May 2010)

Totally, within a period of time of about two months, about 30 ml of *Croton lechleri* resin extract, about 60 ml of *Melaleuca alternifolia* essential oil and about 150 g of ointment of *Calendula Officinalis* were used.

### Example 2 - clinical case of trophic ulcer treated using the kit of the present invention in the presence of Hypericum perforatum as optional elasticiser

A second ulcer present on the same subject of the example 1, was treated starting from 19^{th} March 2010, using the kit of the present invention in the presence of *Hypericum perforatum* as optional elasticiser.

Like in the example 1, the wound was washed using saline solution and it was sprinkled with drops of *Croton lechleri* resin extract i.e. pure *Croton lechleri* resin extracted in 10% of alcohol, leaving the trophic ulcer uncovered, without bandages. A crust had already formed after 12 hours and the re-cicatrisation process had already begun after 24 hours. The strong pains on the leg ceased and the patient stopped using further analgesics.

Drops of *Melaleuca alternifolia* essential oil at 100%, at an amount of drops about twice the amount of *Croton lechleri* extract, were applied around the ulcer thus obtaining an anti-itching lenitive effect, alongside acceleration of the re-epithelization process of the trophic ulcer.

Lastly, some drops of 100% *Hypericum perforatum* essential oil were applied on the re-epithelized part so as to improve the elasticity of the new epithelium.

The two ingredients of the kit of the present invention, as well as the *Hypericum perforatum* essential oil, were applied twice a day, in the morning and in the evening.

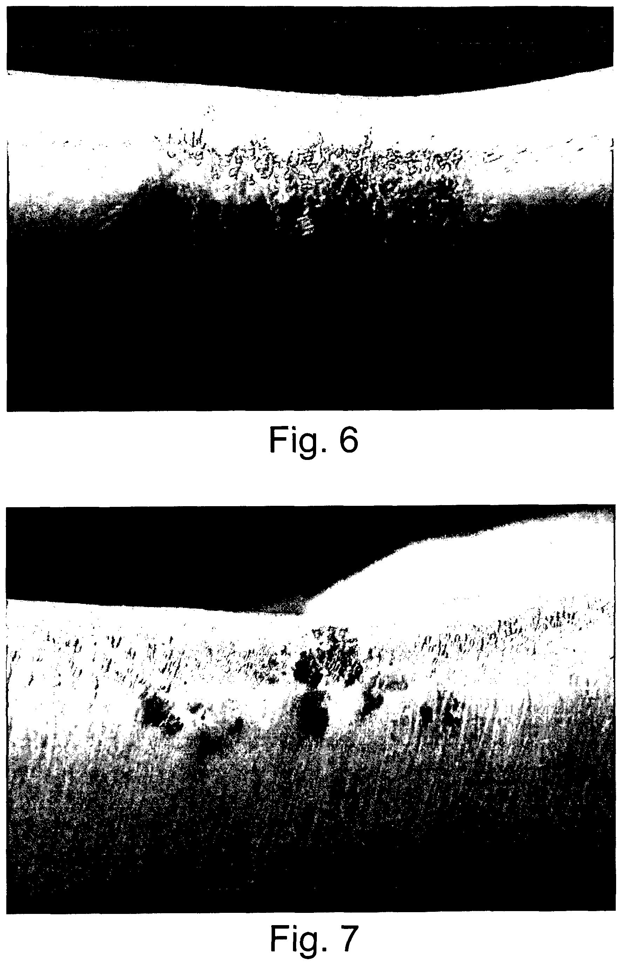

After about five days from the beginning of the treatment, i.e. on 24^{th} March 2010, a crust had formed on the trophic lesion, as shown in Figure 6.

After about nine days from the beginning of the treatment, i.e. on 28^{th} March 2010, the ulcer had considerably reduced (Figure 7).

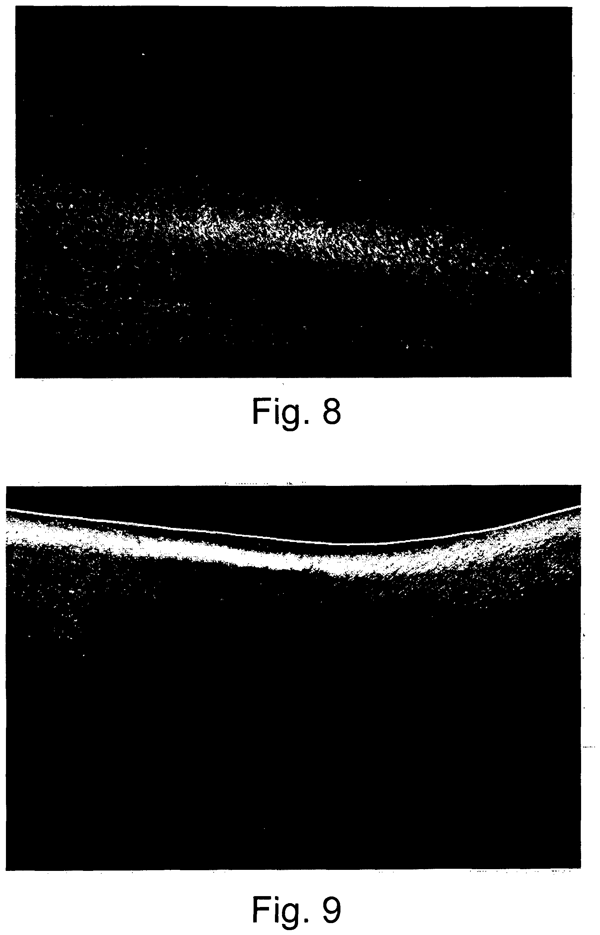

After about 29 days of treatment using the kit of the present invention, alongside the application of *Hypericum perforatum* essential oil, (date 17^{th} April 2010, Figure 8), the extension of the trophic lesion appeared considerably reduced and after 55 days of treatment (13^{th} May 2010, Figure 9), the trophic lesion appeared completely healed.

Totally, within the period of about two months about 10 ml of *Croton lechleri* resin extract, about 20 ml of *Melaleuca alternifolia* essential oil and about 100 ml of *Hypericum perforatum* essential oil were used.

### Example 3 - clinical case of trophic perianal ulcer treated using the kit of the present invention in the presence of Hamamelis virginiana as optional elasticiser

A perianal ulcer present on the same subject of examples 1 and 2, was treated starting from 19^{th} March 2010, using the kit of the present invention in the presence of *Hamamelis virginiana* as optional elasticiser.

Like in the previous examples, the wound was washed using saline solution and it was sprinkled with drops of *Croton lechleri* resin extract i.e. pure *Croton lechleri* resin extracted in 10% of alcohol. A crust had already formed after 12 hours and the re-cicatrisation process had already started after 24 hours.

Drops of *Melaleuca alternifolia* essential oil at 100%, at an amount of drops about twice with respect to the amount of *Croton lechleri* extract, were applied around the ulcer, obtaining an anti-itching lenitive effect, alongside the acceleration of re-epithelization process of the perianal ulcer.

Lastly, a cream based on *Hamamelis virginiana* was applied on the re-epithelized part so as to improve the elasticity of the new epithelium.

The two ingredients of the kit of the present invention, as well as the cream based on *Hamamelis virginiana,* were applied twice a day, in the morning and in the evening, obtaining the complete healing of the perianal ulcer within a period of about two months of treatment.

Totally, within the period of about two months about 10 ml of *Croton lechleri* resin extract, about 30 ml of *Melaleuca alternifolia* essential oil and about 150 g of cream based on *Hamamelis virginiana* were used.

## Claims

1. Kit of parts for use in treating and/or preventing cutaneous ulcers, comprising a *Croton lechleri* resin extract *and Melaleuca alternifolia* oil.

2. Kit of parts for use according to claim 1, further comprising one or more elasticisers.

3. Kit of parts for use according to claim 2, wherein the elasticizer is selected from an ointment based on *Calendula officinalis,* and/or an ointment or cream based on *Hamamelis virginiana,* and/or *Hypericum perforatum* oil.

4. Kit of parts for use according to claim 1, wherein the cutaneous ulcers are chronic ulcers, preferably decubitus ulcers and/or trophic ulcers of the lower limbs or wherein the cutaneous ulcers are ulcers caused by the *Herpes simplex* virus, preferably lip ulcers, or cutaneous ulcers that occur in form of *Herpes zoster.*

5. Kit of parts for use according to claim 1, wherein the *Croton lechleri* resin extract is a liquid solution of resinous latex, 100% of plant origin, of the *Croton Lechleri* plant at least 5 years old and even more preferably it is a liquid solution of pure *Croton lechleri* resin extracted in 10% of alcohol or it is a liquid solution of pure *Croton lechleri* resin in 1:10 dilution in glycerol hydroalcoholic solution, and/or wherein the *Melaleuca alternifolia* oil used is a 100% essential oil.

6. Kit of parts for use according to claim 3, wherein the ointment based on *Calendula officinalis* is a hydroalcoholic extract comprised between 5 and 15% of *Calendula officinalis,* preferably a hydroalcoholic extract at 10% of *Calendula officinalis* and/or wherein the ointment or cream based on *Hamamelis virginiana* contains *Hamamelis virginiana* in a dilution comprised between 1DH and 8DH, preferably between 2DH and 6DH and even more preferably at 4DH and/or wherein the *Hypericum perforatum* oil is a 100% essential oil.

7. Kit of parts for use according to claim 1, wherein the *Croton lechleri* resin extract and the *Melaleuca alternifolia* oil are used at a ratio comprised between 1:1.5 and 1:3, preferably at a ratio comprised between 1:1.6 6 and 1:2.2, and even more preferably the ratio thereof is 1:2.

8. Kit of parts for use according to claim 5, wherein the amount of *Croton lechleri* resin extract administered daily on the ulcers is comprised between 60 and 600 mg, and/or the amount expressed in volume for the daily application on the ulcers, is comprised between 0.20 and 2 ml, preferably between 0.5 and 1.5 ml daily.

9. Kit of parts for use according to claim 1 for use simultaneously, separately or sequentially for treating and/or preventing cutaneous ulcers.

10. Kit of parts for use according to claim 1, wherein the application of the kit of parts on the cutaneous ulcers is performed several times a day, preferably 3-5 times a day, even more preferably 2 times a day, and even more preferably in the morning and in the evening.

11. Kit of parts for use according to claim 1, wherein the active ingredients of the kit of parts are applied on the cutaneous ulcers sequentially, the *Croton lechleri* resin preferably being applied before the *Melaleuca alternifolia* oil.

12. Kit of parts for use according to claim 1, wherein the active ingredients of the kit of parts are applied on the cutaneous ulcers at a time interval between the application of the *Croton lechleri* resin and the application of the *Melaleuca alternifolia* oil, where the time interval is preferably comprised between one day and one week.

13. Kit of parts for use according to claim 1 for treating and/or preventing cutaneous ulcers or wounds in non-human mammals, preferably in felines, canidae or equines.

## Patentansprüche

1. Bestandteile-Bausatz zur Verwendung beim Behandeln und/oder Verhindern von Hautgeschwüren, enthaltend Croton Lechleri (Drachenblut) Harz-Extrakt und Melaleuca Alternifolia (Teebaum) Öl.

2. Bestandteile-Bausatz zur Verwendung nach Anspruch 1, wobei ein oder mehrere Elastikfaktor(en) enthalten ist/sind.

3. Bestandteile-Bausatz zur Verwendung nach Anspruch 2, wobei der Elastikfaktor ausgewählt ist aus einer Salbe basierend auf Calendula Officinalis (Ringelblume) und/oder einer Salbe oder Creme basierend auf Hamamelis Virginiana (virginische Zaubernuss) und/oder Hypericum Perforatum (Johanniskraut) Öl.

4. Bestandteile-Bausatz zur Verwendung nach Anspruch 1, wobei die Hautgeschwüre chronische Geschwüre sind, bevorzugt Dekubitus-Geschwüre und/oder trophische Geschwüre der unteren Gliedmaßen, oder wobei die Hautgeschwüre Geschwüre, die durch den Herpes Simplex Virus verursacht werden, bevorzugt Lippengeschwüre, oder Hautgeschwüre sind, die in der Form von Herpes Zoster auftreten.

5. Bestandteile-Bausatz zur Verwendung nach Anspruch 1, wobei das Croton Lechleri (Drachenblut) Harz-Extrakt eine flüssige Lösung von harzartigem Latex von zu 100 % pflanzlicher Herkunft der zumindest 5 Jahre alten Croton Lechleri (Drachenblut) Pflanze ist und noch bevorzugter es eine flüssige Lösung von reinem Croton Lechleri (Drachenblut) Harz ist, das in 10 % Alkohol extrahiert ist, oder es eine flüssige Lösung von reinem Croton Lechleri (Drachenblut) Harz in einer 1:10 Verdünnung in einer hydroalkoholischen Glycerol-Lösung ist, und/oder wobei das verwendete Melaleuca Alternifolia (Teebaum) Öl ein 100 % ätherisches Öl ist.

6. Bestandteile-Bausatz zur Verwendung nach Anspruch 3, wobei die Salbe basierend auf Calendula Officinalis (Ringelblume) ein hydroalkoholisches Extrakt enthalten zwischen 5 und 15 % an Calendula Officinalis (Ringelblume), bevorzugt ein hydroalkoholisches Extrakt mit 10 % an Calendula Officinalis (Ringelblume), und/oder wobei die Salbe oder Creme basierend auf Hamamelis Virginiana (virginische Zaubernuss) Hamamelis Virginiana (virginische Zaubernuss) in einer Verdünnung enthält, die zwischen 1DH und 8DH, bevorzugt zwischen 2DH und 6DH und noch mehr bevorzugt von 4DH enthalten ist, und/oder wobei das Hypericum Perforatum (Johanniskraut) Öl ein 100 % ätherisches Öl ist.

7. Bestandteile-Bausatz zur Verwendung nach Anspruch 1, wobei der Croton Lechleri (Drachenblut) Harz-Extrakt und das Melaleuca Alternifolia (Teebaum) Öl verwendet werden in einem Verhältnis, das zwischen 1:1,5 und 1:3 enthalten ist, bevorzugt in einem Verhältnis, das zwischen 1:1,6 und 1:2,2 enthalten ist, und noch mehr bevorzugt in dem Verhältnis davon von 1:2.

8. Bestandteile-Bausatz zur Verwendung nach Anspruch 5, wobei die Menge an Croton Lechleri (Drachenblut) Harz-Extrakt, die täglich auf den Geschwüren verabreicht wird, enthalten ist zwischen 60 und 600 mg, und/oder die Menge für die tägliche Anwendung auf den Geschwüren in Volumen ausgedrückt enthalten ist zwischen 0,20 und 2 ml, bevorzugt zwischen 0,5 und 1,5 ml täglich.

9. Bestandteile-Bausatz zur Verwendung nach Anspruch 1 zur Verwendung gleichzeitig, separat oder nacheinander zum Behandeln und/oder Verhindern von Hautgeschwüren.

10. Bestandteile-Bausatz zur Verwendung nach Anspruch 1, wobei die Anwendung des Bestandteile-Bausatzes auf den Hautgeschwüren mehrmals am Tag, bevorzugt 3 - 5 mal am Tag, noch mehr bevorzugt 2 mal am Tag, und noch mehr bevorzugt am Morgen und am Abend ausgeführt wird.

11. Bestandteile-Bausatz zur Verwendung nach Anspruch 1, wobei die aktiven Bestandteile des Bestandteile-Bausatzes nacheinander auf den Hautgeschwüren angewandt werden, wobei das Croton Lechleri (Drachenblut) Harz vor dem Melaleuca Alternifolia (Teebaum) Öl angewendet wird.

12. Bestandteile-Bausatz zur Verwendung nach Anspruch 1, wobei die aktiven Bestandteile des Bestandteile-Bausatzes auf den Hautgeschwüren in einem Zeitintervall zwischen der Anwendung des Croton Lechleri (Drachenblut) Harzes und der Anwendung des Melaleuca Alternifolia (Teebaum) Öls angewandt werden, wobei das Zeitintervall bevorzugt enthalten ist zwischen einem Tag und einer Woche.

13. Bestandteile-Bausatz zur Verwendung nach Anspruch 1 zum Behandeln und/oder Verhindern von Hautgeschwüren oder Wunden bei nicht-menschlichen Säugetieren, bevorzugt katzenartige, hundeartige oder pferdeartige.

## Revendications

1. Kit de produits pour utilisation dans le traitement et/ou la prévention d'ulcères cutanés, comprenant un extrait de résine de *Croton lechleri* et de l'huile de *Melaleuca Alternifolia.*

2. Kit de produits pour utilisation selon la revendication 1, comprenant en outre un ou plusieurs agents assouplissants élastiques.

3. Kit de produits pour utilisation selon la revendication 2, où l'agent assouplissant élastique est choisi parmi une pommade à base de *Calendula Officinalis,* et/ou une pommade ou crème à base de *Hamamelis virginiana,* et/ou de l'huile d'*Hypericum perforatum.*

4. Kit de produits pour utilisation selon la revendication 1, dans lequel les ulcères cutanés sont des ulcères chroniques, de préférence des ulcères de décubitus et/ou des ulcères trophiques des membres inférieurs ou dans lequel les ulcères cutanés sont des ulcères provoqués par le virus *Herpes simplex,* de préférence des ulcères à la lèvre, ou des ulcères cutanés qui se produisent sous la forme d'*Herpes zoster* (zona).

5. Kit de produits pour utilisation selon la revendication 1, dans lequel l'extrait de résine de *Croton lechleri* est une solution liquide de latex résineux, 100% d'origine végétale, de *Croton lechleri* agé d'au moins 5 ans et même plus de préférence, c'est une solution liquide de résine de *Croton lechleri* extraite pure dans 10% d'alcool ou une solution liquide de résine de *Croton lechleri* pure en dilution 1:10 dans une solution hydroalcoolique de glycérol, et/ou dans lequel l'huile de *Melaleuca alternifolia* utilisée est une huile 100% essentielle.

6. Kit de produits pour utilisation selon la revendication 3, dans lequel la pommade à base de *Calendula Officinalis* est un extrait hydroalcoolique comprenant entre 5 et 15% de *Calendula officinalis,* de préférence un extrait hydroalcoolique à 10% de *Calendula officinalis* et/ou dans lequel la pommade ou la crème à base d'*Hamamelis virginiana* contient de *Hamamelis virginiana* dans une dilution comprise entre 1DH et 8DH, de préférence entre 2DH et 6DH et même plus préférentiellement à 4DH et/ou dans lequel l'huile d'*Hypericum perforatum* est une huile 100 % essentielle.

7. Kit de produits pour utilisation selon la revendication 1, dans lequel l'extrait de résine de *Croton lechleri* et l'huile de *Melaleuca alternifolia* sont utilisés dans un rapport compris entre 1:1,5 et 1:3, de préférence dans un rapport compris entre 1:1,6 et 1:2,2, et même plus préférentiellement dans le rapport 1:2.

8. Kit de produits pour utilisation selon la revendication 5, dans lequel la quantité d'extrait de résine de *Croton lechleri* administré quotidiennement sur les ulcères est comprise entre 60 et 600 mg, et/ou la quantité exprimée en volume pour l'application quotidienne sur les ulcères est comprise entre 0,20 et 2 ml, de préférence entre 0,5 et 1,5 ml.

9. Kit de produits pour utilisation selon la revendication 1 pour une utilisation simultanée, séparée ou séquentielle pour traiter et/ou prévenir des ulcères cutanés.

10. Kit de produits pour utilisation selon la revendication 1, dans lequel l'application du kit de produits sur les ulcères cutanés est effectuée plusieurs fois par jour, de préférence 3-5 fois par jour, plus préférentiellement 2 fois par jour, et même encore plus préférentiellement le matin et le soir.

11. Kit de produits pour utilisation selon la revendication 1, dans lequel les substances actives du kit de produits sont appliquées sur les ulcères cutanés séquentiellement, la résine de *Croton lechleri* étant de préférence appliquée avant l'huile de *Melaleuca alternifolia.*

12. Kit de produits pour utilisation selon la revendication 1, dans lequel les substances actives du kit de produits sont appliquées sur les ulcères cutanés selon un intervale de temps entre l'application de la résine de *Croton lechleri* et l'application de l'huile de *Melaleuca alternifolia,* où l'intervale de temps est de préférence compris entre un jour et une semaine.

13. Kit de produits pour utilisation selon la revendication 1 pour le traitement et/ou la prévention d'ulcères ou de blessures cutanés chez les mammifères non-humains, de préférence chez les félins, les canidés ou les équidés.
